(19) **Europäisches Patentamt / European Patent Office / Office européen des brevets**

(11) **EP 3 222 307 B1**

(12) **EUROPEAN PATENT SPECIFICATION**

(45) Date of publication and mention of the grant of the patent:
**28.07.2021  Bulletin 2021/30**

(51) Int Cl.:
***A61M 5/145*** *(2006.01)*

(21) Application number: **16305306.9**

(22) Date of filing: **21.03.2016**

(54) **INFUSION DEVICE FOR ADMINISTERING A MEDICAL FLUID TO A PATIENT**

INFUSIONSVORRICHTUNG ZUR VERABREICHUNG EINES MEDIZINISCHEN FLUIDS AN EINEN PATIENTEN

DISPOSITIF DE PERFUSION PERMETTANT D'ADMINISTRER UN FLUIDE MÉDICAL À UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.09.2017  Bulletin 2017/39**

(73) Proprietor: **Fresenius Vial SAS
38590 Brézins (FR)**

(72) Inventor: **WOLFF, Rémy
38210 Morette (FR)**

(74) Representative: **Fresenius Kabi Deutschland GmbH
Patent Department
Else-Kröner-Straße 1
61352 Bad Homburg (DE)**

(56) References cited:
**WO-A1-2007/094833     WO-A1-2015/123012**

## Description

The invention, which is defined in the independent claim 1, relates to an infusion device for administering a medical fluid to a patient. Prior art infusion devices are known from WO 2015/123012 A1 and WO 2007/094833 A1.

A syringe is received in a receptacle of the infusion device.

Once the syringe is installed on the infusion device, a pusher device of the infusion device is moved using an electric drive device for acting onto a piston of the syringe such that the piston is pushed into a cylindrical tube of the syringe for delivering a medical fluid from the cylindrical tube towards a patient.

During an infusion process, typically the force acting onto the piston is observed by means of a sensing device outputting a sensing signal indicative of the measured force acting in between the pusher device and the piston of the syringe. By observing the force acting onto the piston, the pressure within the syringe and a tubing set connected to the syringe can be monitored such that for example an abnormal pressure condition in particular in case of an occlusion can be detected.

During an infusion process, typically the piston is pushed at a constant velocity into the cylindrical tube of the syringe in order to deliver a medical fluid from the syringe towards a patient at a constant flow rate. The pusher device hence is driven at a constant speed in order to constantly move the piston.

In this regard it represents a challenge to effectively start the infusion process, due to the fact that an unwanted bolus at the beginning of the infusion on the one hand and a long delay time on the other hand beneficially should be avoided.

Generally, an infusion process may be started by moving the pusher device at the targeted pushing velocity corresponding to the flow rate that shall be obtained during the infusion process. In particular for an infusion process at a low flow rate this however may lead to a rather long delay time before the actual delivery of the medical fluid starts, since at the beginning of the movement of the pusher device a (potential) play in between the pusher device and the piston of the syringe as well as an elasticity in the overall syringe pump system and the syringe itself must be overcome before a force can actually be transferred to the piston of the syringe.

It therefore may be beneficial to initially move the pusher device at an increased speed in order to quickly overcome a play and elasticity of the system. Once the pusher device is in functional connection with the piston, it is switched to the targeted pushing velocity such that the actual infusion process takes place at the targeted velocity. Because it however may not reliably be known when the play between the pusher device and the piston as well as the elasticity of the system is overcome, this approach is prone to the risk that at the start of the infusion process an uncontrolled volume (bolus) is infused to the patient, which may be detrimental to the patient's health.

Nowadays, infusion devices exist in which a so called "quickstart" feature is implemented. In such an infusion device, the pusher device initially is moved at an increased velocity until it can be assumed that the further movement of the pusher device will cause a movement of the piston of the syringe such that the medical fluid is delivered from the syringe towards the patient. Commonly herein, the force acting onto the piston is observed, and if the force exceeds a predetermined maximum force or if the distance by which the pusher device has been moved exceeds a predetermined maximum distance, it is assumed that delivery of the medical fluid from the syringe can start. This approach however may not be very efficient, because for example a predetermined maximum distance could be reached before the delivery of the medical fluid from the syringe actually starts, or the comparison of the force with the predetermined maximum force may not provide for an accurate estimation whether infusion is about to start or not, depending in particular also on the type of syringe used. In addition, if a syringe is installed on an infusion device and an infusion process is not immediately started, but only after a significant waiting time, the piston of the syringe may stick to the cylindrical tube, rendering the interruption of the quickstart procedure according to a force criteria even less reliable.

It is an object of the instant invention to provide an infusion device for administering a medical fluid to a patient, the infusion device allowing for an efficient quickstart procedure.

Accordingly, it is provided that, at the beginning of moving the pusher device for acting onto the piston, a startup routine is executed in which

- in a first phase the electric drive device drives the pusher device to move at a first velocity, and
- if by means of the sensing signal a drop in the measured force acting in between the pusher device and the piston of the syringe is observed, the electric drive device is controlled to drive the pusher device to move at a second velocity different than the first velocity.

The instant invention starts up from the finding that at the beginning of the actual movement of the piston of the syringe a drop in force may occur, the drop in force very reliably and accurately indicating that the piston starts moving. Typically, when a pusher device is moved at the start up of an infusion process, once the pusher device acts onto the piston the force (initially) will rise, the movement of the pusher device however not yet causing a movement of the piston. In this phase, an elasticity of the infusion pump system as well as of the syringe is overcome until an efficient force transfer onto the piston can take place. Once the piston starts to move together with the pusher device, the force measured by the sensing device will drop by at least some degree and will converge towards the force needed to move the piston within the cylindrical tube for delivering the medical fluid from the cylindrical tube of the syringe towards the patient. The drop in force is due to a transition from a sticking friction of the piston within the cylindrical tube of the syringe towards a sliding friction. Once the (sticking) piston is released and begins to move, the force acting onto the piston is reduced, because the force required for moving the piston is smaller than the force required to overcome the initial sticking (static) friction.

Hence, by observing a drop in the measured force, it can be reliably and accurately determined when the piston begins to move and, hence, at what point in time the delivery of the medical fluid from the syringe towards the patient starts. According to the drop in the measured force it hence can be precisely switched between an initial startup routine in which the pusher device is driven at an initial, first velocity and the actual delivery operation at which the pusher device is driven at a second, different velocity corresponding to the targeted flow rate which shall be achieved for delivering the medical fluid towards the patient.

The first velocity may in particular be larger than the second velocity. During the startup routine the pusher device hence can be quickly brought into operative connection with the piston such that the delivery of the medical fluid from the syringe can start. During the initial startup routine, a play in between the pusher device and the piston and an elasticity in the infusion pump system as well as in the syringe are overcome such that an effective force transfer from the pusher device to the piston can be achieved.

The drop in the force may be for example observed by comparing force readings at different times. If for example it is found that a force reading at a particular instance of time is smaller than the force reading at a previous instance of time, it is found that the force has dropped. Herein, an averaging over multiple force readings may take place. Alternatively or in addition, the drop in force may be checked for significance by comparing the drop in force to a predefined margin such that a drop in force is only concluded if the drop is larger than the predefined margin.

After the drive device is switched to drive the pusher device at the second velocity, delivery of the medical fluid from the syringe starts. The second velocity herein may be constant and may correspond to a constant targeted flow rate which shall be obtained for the infusion process. It however is also conceivable that the targeted flow rate varies overtime and hence is not constant.

The first velocity beneficially is larger than the second velocity. The first velocity herein beneficially is chosen large enough such that a large delay until delivery starts is avoided, at the same time however small enough such that a reliable and quick switching of velocities is possible once a drop in force is detected, without a substantial risk for an overshoot which otherwise could cause an unwanted bolus.

Situations may occur in which a drop in force cannot (reliably) be detected. In those situations it may be advantageous to switch from the first velocity to the second velocity making use of a different criteria. For example, the electric drive device may be controlled to drive the pusher device to move at the second velocity if the pusher device has been moved during the first phase over a distance exceeding a predetermined maximum traveling distance. If hence the pusher device has been moved at the first velocity by a maximum allowed distance, it is switched to the second velocity. Alternatively, it may be switched to the second velocity if a measured force is observed to exceed a predefined maximum force. If hence the measured force rises beyond a permissible bound, it also is switched to the second velocity. Even if no drop in force is detected, the startup routine hence is terminated and it is switched to the second velocity (corresponding to the targeted flow rate for the actual infusion process).

In the latter case the predefined maximum force may be adjusted depending on for example the time in between the installation of the syringe in the receptacle of the infusion device and the beginning of the infusion process. This is based on the finding that the force required to release the piston from its sticking friction within the cylindrical tube increases the longer the piston rests within the cylindrical tube prior to the infusion process. If the syringe is installed on the infusion device and it then is waited for a rather long time (for example a few hours) until the infusion process starts, the piston will stick to the cylindrical tube of the syringe at a rather large sticking friction. This can be taken into account by increasing the maximum force used in the force criteria. The maximum force may for example be computed by multiplying a constant force value with a factor depending on the waiting time between the installation of the syringe in the receptacle and the beginning of moving the pusher device.

The object is achieved by an infusion device for administering a medical fluid to a patient, as defined in the independent claim 1.

The idea underlying the invention shall subsequently be described in more detail with respect to the embodiments shown in the figures. Herein:

Fig. 1 shows a view of an embodiment of an infusion device in the shape of a syringe pump;

Fig. 2 shows a schematic view of a drive mechanism of the infusion device;

Fig. 3 shows a schematic view of a clutching device of a drive element of the drive mechanism;

Fig. 4 a schematic, enlarged view of a syringe;

Fig. 5A a graphical drawing a measured force over the distance travelled by the pusher device; and

Fig. 5B a graphical drawing of the velocity of the pusher device over distance.

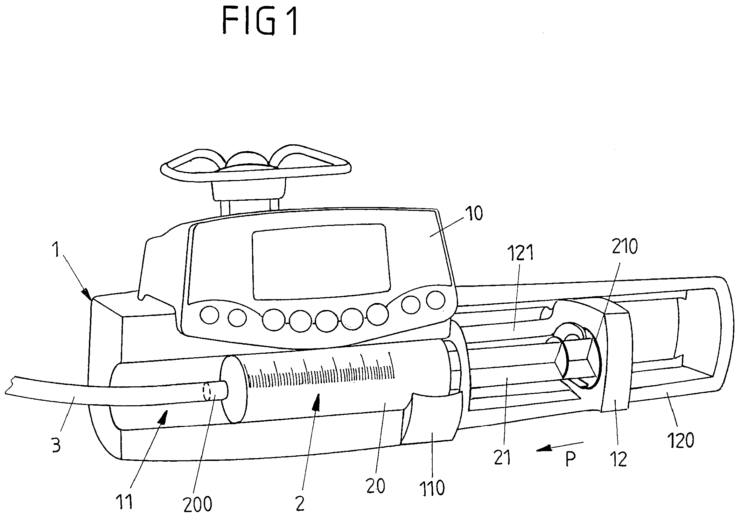

Fig. 1 shows an infusion device 1 in the shape of a syringe pump having a housing 10 and a receptacle 11 arranged on the housing 10 to receive a syringe 2 therein.

The syringe 2 comprises a cylindrical tube 20 which, when installing the syringe 2 on the infusion device 1, contains a medical liquid, for example a medication or a solution for the parenteral feeding, to be infused to a patient. The cylindrical tube 20 is connected, via a connector 200, to an infusion line 3 which may extend from the syringe 2 towards a patient for infusing the medical liquid to the patient.

For installing the syringe 2 on the receptacle 11 of the infusion device 1, the cylindrical tube 20 of the syringe 2 is placed in the receptacle 11 and is mechanically connected to the housing 10 by means of a fixation device 110. By means of the fixation device 110, for example constituted by a releasable clamp element, the cylindrical tube 20 is secured within the receptacle 11 such that the cylindrical tube 20 is held in position on the receptacle 11.

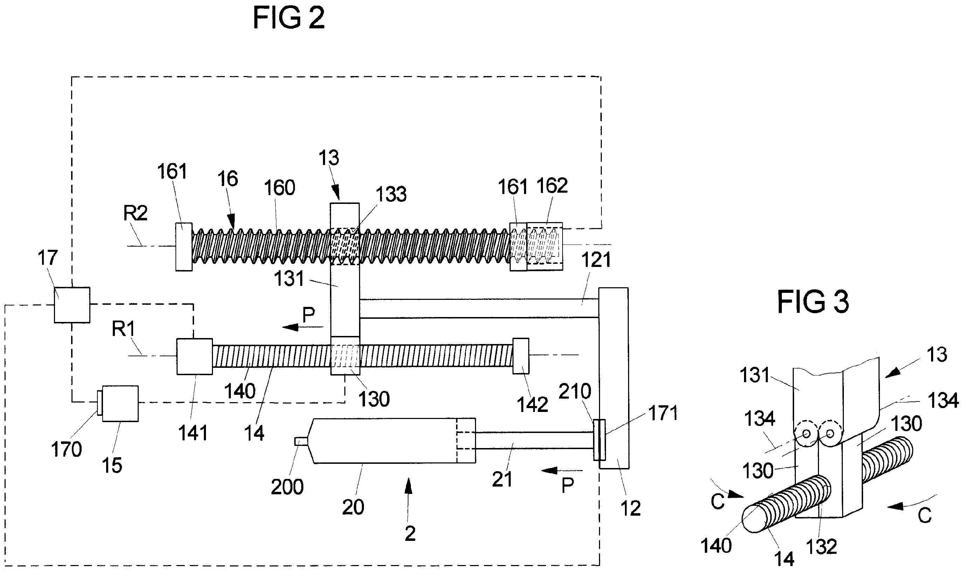

The syringe 2 comprises a piston 21 which, for delivering medical fluid contained in the cylindrical tube 20, can be pushed into the cylindrical tube 20 in a pushing direction P. For this, the infusion device 1 comprises a pusher device 12 movably arranged within a guide device 120 and connected to a drive mechanism (which subsequently shall be described with relation to Fig. 2 and 3) via a connecting rod 121.

For operating the infusion device 1, the syringe 2 is installed on the infusion device 1 and the pusher device 12 is (manually) moved towards a piston head 210 of the piston 21 until the pusher device 12 comes into abutment with the piston head 210. For performing an infusion process the pusher device 12 is then electrically moved in the pushing direction P to move the piston 21 into the cylindrical tube 20 for delivering the medical fluid contained in the cylindrical tube 20 via the infusion line 3 towards the patient.

The pusher device 12 is driven by a drive mechanism, which, according to one embodiment, is schematically illustrated in Fig. 2. The drive mechanism comprises a drive element 13, which is connected to the pusher device 12 we a connecting rod 121 in a mechanically fixed manner such that by moving the drive element 13 the pusher device 12 is moved along the pushing direction P. The drive element 13 is movable within the housing 10 along the pushing direction P and, via a clutching device 130, is (releasably) connected to a driving rod 14 having a screw thread 140.

A schematic view of an embodiment of the clutching device 130 is shown in Fig. 3. The clutching device comprises two clutch elements 130 which each are pivotably connected, about an associated pivot axis 134, to a frame member 131 of the drive element 13. The clutch elements 130 each are shaped as a half nut and comprise a screw thread 132 by which they may engage with the screw thread 140 of the driving rod 14.

In a clutched state, as illustrated in Fig. 3, the clutch elements 130 are pivoted towards each other in a clutching direction C such that they receive the driving rod 14 in between them and engage with the screw thread 140 of the driving rod 14. To release the engagement, the clutch elements 130 are pivoted opposite to the clutching direction C away from one another, such that they disengage from the screw thread 140 of the driving rod 14 and hence release the connection between the drive element 13 and the driving rod 14.

During regular infusion operation of the infusion device 1 the clutch device is in the clutched state in which the clutch elements 130 engage with the screw thread 140 of the driving rod 14. The driving rod 14, at one end, is connected to an electric drive motor 141 and at the other end is received in a bearing 142 such that, driven by the electric drive motor 141, the driving rod 14 can be rotated about an axis of rotation R1. By rotating the driving rod 14, the drive element 13 (due to the engagement of the clutch elements 130 with the screw thread 140 of the driving rod 14) is longitudinally moved along the driving rod 14, and by the movement of the drive element 13 the pusher device 12 pushes the piston 21 in the pushing direction P into the cylindrical tube 20 of the syringe 2.

The drive element 13 is operatively connected to a brake device 16 having a threaded spindle 160 which is rotatable, within bearings 161, about a rotational axis R2. The drive element 13 comprises an engagement opening 133 having a screw thread therein which engages with a screw thread of the threaded spindle 160. A longitudinal movement of the drive element 13 along the pushing direction P hence causes, due to the engagement, the threaded spindle 160 to be rotated about the rotational axis R2, which generally may be possible at low force if the screw thread of the threaded spindle 160 has a comparatively large pitch.

The threaded spindle 160, at one end, is associated with a brake 162 constituted for example by an electromagnetic brake. If the brake 162 is activated, it blocks a rotation of the threaded spindle 160 about its rotational axis R2. If the threaded spindle 160 is not able to rotate, the drive element 13 cannot move along the threaded spindle 160 such that the drive element 13 is held in position and hence is braked by the brake device 16. If the brake 162 in contrast is deactivated, the threaded spindle 160 is allowed to rotate, such that the drive element 13 is not braked and may be moved longitudinally along the threaded spindle 160.

The operation of the infusion device 1 is controlled by means of a control device 17. In particular, the control device 17 acts onto the electric drive device 141 to rotate the driving rod 14, and the control device 17 acts onto the brake 162 to switch the brake device 16 between its activated and its deactivated state.

The clutch device implemented by the clutch elements 130 is actuatable by means of an actuation device 15, for example in the shape of a lever. Herein, the lever may be manually pressed to unclutch the clutch elements 130 from the driving rod 14, and may be released in order to revert the clutching device to its clutched state.

The drive mechanism as schematically illustrated in Fig. 2 and 3 may, in one embodiment, be implemented by a mechanism as it is described in US 2012/015170 A1, which shall be incorporated by reference herein.

In addition, sensing devices 170, 171 are provided which serve to monitor an actuation state of the actuation device 15 and a force between the pusher device 12 and the piston head 210 of the piston 21.

In particular, a first sensing device 170 detects whether the clutching device is actuated to its unclutched state or its clutched state. The first sensing device 170 can for example be implemented by a mechanical switch, for example a micromechanical switch, for monitoring a position of a lever of the actuation device 15.

A second sensing device 171 is implemented by a force sensor, such as piezoelectric sensor.

The sensing devices 170, 171 both issue sensor signals which are fed to the control device 17 and may be used for controlling the operation of the infusion device 1.

As said, for installing a syringe 2 on the infusion device 1 the pusher device 12 is manually moved such that it comes into contact with the piston 21. For this, the clutching device is activated by means of the activation device 15 to its unclutched state, such that the drive element 13 can freely be moved along the driving rod 14. Once the pusher device 12 has come into contact with the piston head 210 of the piston 21, the clutching device is manually brought into engagement with the driving rod 14 by releasing the lever of the actuation device 15.

In addition, during operation of the infusion device it is conceivable that a user may want to perform a manual bolus by manually pushing the pusher device 12 in the pushing direction P to push the piston 21 into the cylindrical tube 20. For this, the clutching device is unclutched such that the pusher device 12 is manually movable, and, after the manual bolus has been performed, is manually reverted to its clutched state such that the regular infusion operation may resume.

At the beginning of an infusion operation, or when resuming an infusion operation after performing a manual bolus, the pusher device 12 is moved in the pushing direction P towards the piston head 210, wherein initially a remaining play in between the pusher device 12 and the piston head 210 may exist and an elasticity in the overall system must be overcome before the moving of the pusher device 12 actually leads to a movement of the piston 21 within the cylindrical tube 20. Hence, at the beginning of the infusion operation a movement of the pusher device 12 will not immediately cause a movement of the piston 21, such that the actual delivery of the medical fluid from the cylindrical tube 20 is delayed.

In order to keep the delay as short as possible and in order to at the same time avoid the risk for an unwanted bolus at the beginning of the infusion operation, a startup routine is performed in which, initially, the pusher device 12 is moved towards the piston 21 at an increased, first velocity until the pusher device 12 comes into operational connection with the piston head 210 of the piston 21 and delivery of the medical fluid can start. Once it is found that delivery can start, it is switched to a second velocity and the pusher device 12 from now on is pushed with said second velocity which corresponds to a targeted flow rate at which the delivery of the medical fluid from the syringe 2 towards the patient shall take place.

It is a challenging task to detect the instance at which delivery starts, i.e., at which a (further) movement of the pusher device 12 in the pushing direction P will cause a movement of the piston 21 and hence a delivery of medical fluid from the cylindrical tube 20 towards the patient.

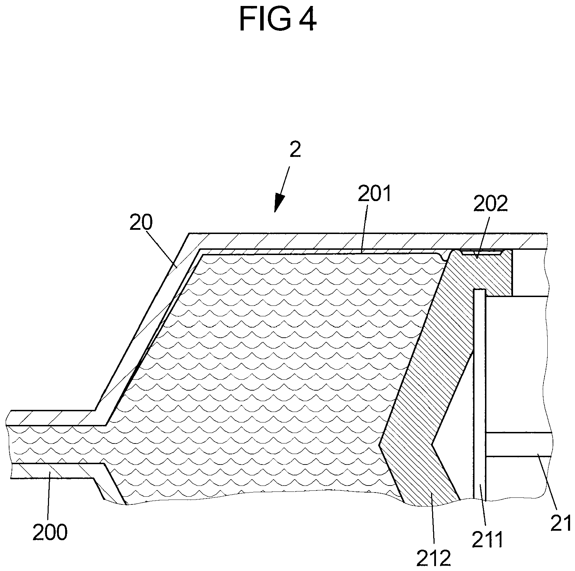

The piston 21, as schematically indicated in Fig. 4, rests with a syringe stopper 212 (for example made out of rubber) within the cylindrical tube 20 and is in close contact with an inner wall 202 of the cylindrical tube 20. Generally, a grease film 201 may be covering the inner wall 202 of the cylindrical tube 20, the grease film 201 having an influence on the friction in-between the syringe stopper 212 and the inner wall 202 of the cylindrical tube 20.

In particular if the piston 21 has rested for a significant time within the cylindrical tube 20 without being moved, the piston 21 with its syringe stopper 212 will stick to the inner wall 202 of the cylindrical tube 20. The sticking is due to a sticking (static) friction acting in-between the syringe stopper 212 and the inner wall 202 of the cylindrical tube 20 (made for example of polyethylene), which needs to be overcome in order to move the piston 21 within the cylindrical tube 20.

Generally, the sliding friction occurring when moving the piston 21 relative to the cylindrical tube 20 is smaller than the sticking (static) friction. Hence, to overcome the sticking friction a larger force is required than for afterwards moving the piston 21 relative to the cylindrical tube 20.

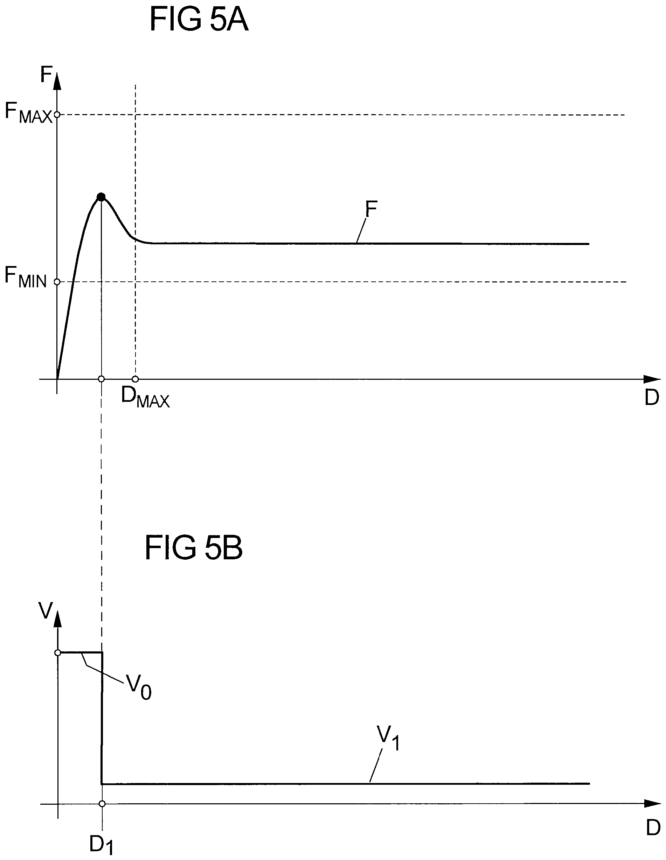

This can be observed also in the force F acting in between the pusher device 12 and the piston 21 at the start of an infusion operation, as illustrated in Fig. 5A. At the start of an infusion operation, the force F first rises, due to an elasticity in the system, without the movement of the pusher device 12 actually causing a movement of the piston 21. The force F keeps rising until a peak is observed after which the force F drops and converges towards the force F actually needed to slidingly move the piston 21 within the cylindrical tube 20. At the location of the peak the sticking friction of the piston 21 is overcome and the piston 21 starts to move relative to the cylindrical tube 20, causing the force F to drop.

The peak of the force F hence rather precisely indicates the point in time at which the piston 21 starts moving and delivery of the medical fluid begins. By detecting the peak it thus can be determined when the delivery of the medical fluid actually starts.

Accordingly, at the beginning of an infusion process the pusher device 12 is driven at a first velocity VO, as illustrated in the velocity curve V of Fig. 5B. The first velocity VO is chosen to be rather large in order to quickly bring the pusher device 12 in operative connection with the piston 21 and to quickly overcome a play in between the pusher device 12 and the piston 21 and the elasticity of the system. During this initial, first phase the force F, as visible in Fig. 5A, continuously rises, which is observed by force readings taken by the sensing device 171 measuring the force F acting in between the pusher device 12 and the piston 21.

Once a drop in force F is detected, the drive device 141 is controlled to drive the pusher device 12 at a second, smaller velocity V1, as illustrated in Fig. 5B. This second velocity V1 corresponds to the targeted flow rate which shall be used to deliver a medical fluid from the syringe 2 towards the patient.

The drop in force F is detected for example by comparing consecutive force values. If it is found that the force value at a particular instance of time is smaller than the force value at a previous instance of time, it is concluded that the force F has dropped, causing to switch from the first velocity VO to the second velocity V1. Herein, an averaging may take place, and/or it may be checked whether the drop exceeds a predetermined margin in order to exclude effects from a noisy signal or the like.

By observing whether a drop in force F occurs, it can precisely be determined at which point in time the piston 21 starts moving. At this point in time it is switched to the second velocity V1 such that the pusher device 12 and - together with the pusher device 12 the piston 21 - is moved at the second velocity V1 corresponding to the targeted, desired flow rate for the delivery of the medical fluid from the syringe 2 towards the patient.

Situations may occur in which no drop in force F can be detected. In this case it may be switched to the second velocity V1 if the pusher device 12, during the initial, first phase, has been moved by a distance D exceeding a predefined maximum distance DMAX. Alternatively, it may be switched to the second velocity V1 if the measured force F exceeds a predetermined maximum force FMAX. Hence, if no drop in force F is detected, at some point it is switched to the second, smaller velocity V1 for driving the pusher device 12 and, with it, the piston 21.

It also is conceivable that, if a risk for an initial bolus shall under all circumstances be avoided, it already is switched to the second velocity V1 if a predefined minimum force FMIN is exceeded. By using the minimum force FMIN in a force criteria and by choosing the minimum force FMIN substantially smaller than the maximum force FMAX it can be made sure that it is guaranteed that no bolus can occur at the beginning of the infusion operation.

Whether the minimum force FMIN is used in a criteria to determine when to switch from the first velocity V0 to the second velocity V1, or whether the switch in velocities is initiated upon observing a drop in force F (or alternatively upon exceeding the maximum force FMAX), may be a user selectable configuration setting. Namely, an infusion device may allow a setting "bolus preferred" or, alternatively, a setting "no bolus preferred".

If the setting "bolus preferred" is chosen, the switch from the first velocity VO to the second velocity V1 is triggered when observing a drop in force, as described above, or, if no drop in force is observed, once the maximum distance DMAX or the maximum force FMAX is exceeded.

In this case it is likely that the switch in velocities takes place simultaneously with or after the piston 21 starts to move and hence the infusion process starts. A (small) bolus may occur, which however may be acceptable. This option allows for a start of the infusion process with only a small delay, because the pusher device 12 is approached towards the piston 21 until the piston 21 actually is unstuck and hence starts to move.

This option may for example be chosen in a channel relay procedure (for example used for catecholamine administration).

If alternatively the setting "no bolus preferred" is chosen, the switch from the first velocity VO to the second velocity V1 is triggered once the measured force F exceeds the minimum force FMIN.

In this case it is made sure that the switch in velocities takes place prior to the piston 21 starting to move and hence prior to the start of the actual infusion. This option may be chosen if a bolus at the beginning of the infusion operation must be avoided. This option comes at the expense of an increased delay prior to the actual start of the infusion operation, because it is switched to the smaller, second velocity V1 at an earlier point in time and it hence takes longer to start the actual infusion operation.

An infusion device 1 may be configured to choose automatically between the setting "bolus preferred" and the setting "no bolus preferred". For example, the infusion device 1 may be configured to automatically choose the option "bolus preferred" in case of a channel relay procedure and the option "no bolus preferred" in all other cases. A user may have the option to adapt the setting, if desired.

Generally, the sticking friction by which the piston 21 is held in place within the cylindrical tube 20 depends on the amount of time the piston 21 rests unmoved within the cylindrical tube 20. Generally, if the waiting time between the installation of the syringe 2 on the infusion device 1 and the actual start of the infusion operation is large, the sticking friction will be large. This can be taken into account for example for determining the maximum permissible force FMAX by computing the maximum force FMAX using an equation such as:

$$FMAX = F_{Max,\ constant} \cdot K(\text{waiting time})$$

Likewise, the minimum force FMIN can be determined using the equation

$$FMIN = F_{Min,\ constant} \cdot K(\text{waiting time})$$

Herein, $F_{Max,\ constant}$ and $F_{Min,\ constant}$ represent constant values and K(waiting time) is a coefficient depending on the waiting time and having values for example in the range between 1 and 1.3 varying with the waiting time. The waiting time may for example range from a few minutes to a few hours.

By choosing the maximum force respectively the minimum force dependent on the waiting time, the effect of a varying sticking friction is taken into account for the force criteria which applies if no drop in force is detected.

The startup routine of the described kind allows to quickly approach the pusher device 12 towards the piston 21, with a small risk of an unwanted initial bolus at the start of an infusion process. Herein, the first velocity VO during the first phase of the startup routine is chosen large enough such that a delay at the beginning of the infusion operation is kept small, at the same time however small enough such that a switch between the velocities V0, V1 is quickly and effectively possible without causing a significant bolus.

The idea underlying the invention is not limited to the embodiments described above.

In particular, the infusion device may have a different shape and mechanical setup than described above.

The proposed startup routine can be used on syringes of different types and different volumes. Herein, depending on the syringe type and syringe volume the maximum and minimum force as well as the maximum distance may vary.

**List of Reference Numerals**

| | |
|---|---|
| 1 | Infusion device |
| 10 | Housing |
| 11 | Receptacle |
| 110 | Fixation device |
| 12 | Pusher device |
| 120 | Guide device |
| 121 | Connecting rod |
| 13 | Drive element |
| 130 | Clutch element |
| 131 | Frame member |
| 132 | Screw thread |
| 133 | Engagement opening |
| 134 | Pivot axis |
| 14 | Driving rod (spindle) |
| 140 | Screw thread |
| 141 | Drive motor |
| 142 | Bearing |
| 15 | Actuation device (handle) |
| 16 | Brake device |
| 160 | Threaded spindle |
| 161 | Bearing |
| 162 | Brake |
| 17 | Control device |
| 170, 171 | Sensing device |
| 2 | Syringe |
| 20 | Cylinder tube |
| 200 | Connector |
| 201 | Grease film |
| 202 | Inner wall |
| 21 | Piston |
| 210 | Piston head |
| 211 | Flange |
| 212 | Syringe stopper |
| 3 | Infusion line |
| C | Clutching direction |
| D | Distance |
| DMAX | Maximum distance |
| F | Force |
| FMAX | Maximum force |
| FMIN | Minimum force |
| P | Pushing direction |
| R1, R2 | Axis of rotation |
| V | Velocity |
| VO | Startup velocity |
| V1 | Targeted flow rate velocity |

**Claims**

1.  Infusion device (1) for administering a medical fluid to a patient, comprising:

    - a receptacle (11) for receiving a syringe (2),
    - a pusher device (12) movable in a pushing direction (P) for acting onto a piston (21) of a syringe (2) received in the receptacle (11),
    - an electric drive device (141) for moving the pusher device (12),
    - a sensing device (171) constituted to output a sensing signal indicative of a force (F) acting in between the pusher device (12) and the piston (21) of the syringe (2), and
    - a control device (17) for controlling the electric drive device (141) for moving the pusher device (12),

    wherein the control device (17) is constituted to execute, at the beginning of moving the pusher device (12) for acting onto the piston (21), a startup routine in which

  - in a first phase the electric drive device (141) drives the pusher device (12) to move at a first velocity (V0), and
  - if by means of the sensing signal a drop in the measured force (F) acting in between the pusher device (12) and the piston (21) of the syringe (2) is observed, the electric drive device (141) is switched to drive the pusher device (12) to move at a second velocity (V1) different than the first velocity (V0),

  **characterized in**
  **that** the control device (17) is constituted to control the electric drive device (141) to drive the pusher device (12) to move at the second velocity (V1) if a measured force (F) is observed exceeding a predefined maximum force (FMAX), even if no drop in the force (F) is detected, wherein the predefined maximum force (FMAX) is determined in dependence on the time in between the installation of the syringe (2) in the receptacle (11) and the beginning of moving the pusher device (12).

2. Infusion device (1) according to claim 1, **characterized in that** a drop in the force (F) is present if the sensing signal indicates that the force at a particular instance of time ($F_i$) is smaller than the force at a previous instance of time ($F_{i-1}$).

3. Infusion device (1) according to claim 1 or 2, **characterized in that** the second velocity (V1) is smaller than the first velocity (V0).

4. Infusion device (1) according to one of claims 1 to 3, **characterized in that** the second velocity (V1) corresponds to a targeted flow rate for the infusion process.

5. Infusion device (1) according to one of claims 1 to 4, **characterized in that** the first velocity (V0) lies in the range between 0.05mm/s to 2.5mm/s, for example in between 0.1mm/s to 0.15mm/s.

**Patentansprüche**

1. Infusionsvorrichtung (1) zum Verabreichen eines medizinischen Fluids an einen Patienten, umfassend:

  - ein Behältnis (11) zum Aufnehmen einer Spritze (2),
  - eine Schubvorrichtung (12), die in eine Schubrichtung (P) beweglich ist, um auf einen Kolben (21) einzuwirken einer Spritze (2), die in dem Behältnis (11) aufgenommen ist,
  - eine elektrische Antriebsvorrichtung (141) zum Bewegen der Schubvorrichtung (12),
  - eine Erfassungsvorrichtung (171), die konzipiert ist, um ein Erfassungssignal auszugeben, das eine Kraft (F) angibt, die zwischen der Schubvorrichtung (12) und dem Kolben (21) der Spritze (2) wirkt und
  - eine Steuervorrichtung (17) zum Steuern der elektrischen Antriebsvorrichtung (141) zum Bewegen der Schubvorrichtung (12),

  wobei die Steuervorrichtung (17) konzipiert ist, um, zu Beginn der Bewegung der Schubvorrichtung (12) zum Wirken auf den Kolben (21), eine Startroutine durchzuführen, wobei

  - in einer ersten Phase die elektrische Antriebsvorrichtung (141) die Schubvorrichtung (12) antreibt, um sich mit einer ersten Geschwindigkeit (V0) zu bewegen, und
  - falls mittels des Erfassungssignals ein Abfall in der gemessenen Kraft (F), die zwischen der Schubvorrichtung (12) und dem Kolben (21) der Spritze (2) wirkt, beobachtet wird, die elektrische Antriebsvorrichtung (141) geschaltet wird, um die Schubvorrichtung (12) anzutreiben, um sich mit einer zweiten von der ersten Geschwindigkeit (V0) verschiedenen Geschwindigkeit (V1) zu bewegen,

  **dadurch gekennzeichnet, dass**
  die Steuervorrichtung (17) konzipiert ist, um die elektrische Antriebsvorrichtung (141) zu steuern, um die Schubvorrichtung (12) mit einer zweiten Geschwindigkeit (V1) zu bewegen, falls eine gemessene Kraft (F) beobachtet wird, die eine vordefinierte maximale Kraft (FMAX) überschreitet, sogar wenn kein Abfall in der Kraft (F) erkannt wird, wobei die vordefinierte maximale Kraft (FMAX) in Abhängigkeit von der Zeit zwischen der Installation der Spritze (2) in dem Behältnis (11) und dem Beginn der Bewegung der Schubvorrichtung (12) bestimmt wird.

2. Infusionsvorrichtung (1) nach Anspruch 1, **dadurch gekennzeichnet, dass** ein Abfall in der Kraft (F) vorhanden ist, wenn das Erfassungssignal angibt, dass die Kraft zu einem bestimmten Zeitpunkt ($F_i$) kleiner ist als die Kraft zu einem vorherigen Zeitpunkt ($F_{i-1}$).

3. Infusionsvorrichtung (1) nach Anspruch 1 oder 2, **dadurch gekennzeichnet, dass** die zweite Geschwindigkeit (V1) kleiner als die erste Geschwindigkeit (V0) ist.

4. Infusionsvorrichtung (1) nach einem der Ansprüche 1 bis 3, **dadurch gekennzeichnet, dass** die zweite Geschwindigkeit (V1) einer angezielten Durchflussrate für den Infusionsprozess entspricht.

5. Infusionsvorrichtung (1) nach einem der Ansprüche 1 bis 4, **dadurch gekennzeichnet, dass** die erste Geschwindigkeit (V0) in dem Bereich zwischen 0,05 mm/s bis 2,5 mm/s liegt, zum Beispiel zwischen 0,1 mm/s bis 0,15 mm/s.

**Revendications**

1. Dispositif de perfusion (1) pour administrer un fluide médical à un patient, comprenant :

   - un réceptacle (11) pour recevoir une seringue (2),
   - un dispositif poussoir (12) mobile dans une direction de poussée (P) pour agir sur un piston (21) d'une seringue (2) reçue dans le réceptacle (11),
   - un dispositif d'entraînement électrique (141) pour déplacer le dispositif poussoir (12),
   - un dispositif de détection (171) constitué pour délivrer un signal de détection indicatif d'une force (F) agissant entre le dispositif poussoir (12) et le piston (21) de la seringue (2), et
   - un dispositif de contrôle (17) pour contrôler le dispositif d'entraînement électrique (141) pour déplacer le dispositif poussoir (12),

   dans lequel le dispositif de contrôle (17) est constitué pour exécuter, au début du déplacement du dispositif poussoir (12) pour agir sur le piston (21), une routine de démarrage dans laquelle

   - dans une première phase le dispositif d'entraînement électrique (141) entraîne le dispositif poussoir (12) à se déplacer à une première vitesse (V0), et
   - si au moyen du signal de détection une chute de la force mesurée (F) agissant entre le dispositif poussoir (12) et le piston (21) de la seringue (2) est observée, le dispositif d'entraînement électrique (141) est commuté pour entraîner le dispositif poussoir (12) à se déplacer à une seconde vitesse (V1) différente de la première vitesse (V0),

   **caractérisé en ce que** le dispositif de contrôle (17) est constitué pour contrôler le dispositif d'entraînement électrique (141) pour entraîner le dispositif poussoir (12) à se déplacer à la seconde vitesse (V1) si une force mesurée (F) est observée dépassant une force maximale prédéfinie (FMAX), même si aucune baisse de la force (F) n'est détectée, dans lequel la force maximale prédéfinie (FMAX) est déterminée en fonction du temps entre l'installation de la seringue (2) dans le réceptacle (11) et le début du déplacement du dispositif poussoir (12).

2. Dispositif de perfusion (1) selon la revendication 1, **caractérisé en ce qu'**une chute de la force (F) est présente si le signal de détection indique que la force à un instant de temps particulier ($F_i$) est inférieure à la force à un instant de temps précédent ($F_{i-1}$).

3. Dispositif de perfusion (1) selon la revendication 1 ou 2, **caractérisé en ce que** la seconde vitesse (V1) est inférieure à la première vitesse (V0).

4. Dispositif de perfusion (1) selon l'une des revendications 1 à 3, **caractérisé en ce que** la seconde vitesse (V1) correspond à un débit cible pour le processus de perfusion.

5. Dispositif de perfusion (1) selon l'une des revendications 1 à 4, **caractérisé en ce que** la première vitesse (V0) est comprise dans la plage entre 0,05 mm/s et 2,5 mm/s, par exemple entre 0,1 mm/s et 0,15 mm/s.

FIG1

FIG 2

FIG 3

EP 3 222 307 B1

# FIG 4

## FIG 5A

## FIG 5B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015123012 A1
- WO 2007094833 A1
- US 2012015170 A1